(19)

Europäisches
Patentamt

European
Patent Office

Office européen
des brevets

(11) **EP 4 610 994 A1**

(12) **EUROPEAN PATENT APPLICATION**

(43) Date of publication:
**03.09.2025 Bulletin 2025/36**

(21) Application number: **24160294.5**

(22) Date of filing: **28.02.2024**

(51) International Patent Classification (IPC):
**G16H 20/17** (2018.01) **G16H 50/20** (2018.01)

(52) Cooperative Patent Classification (CPC):
**G16H 20/17; G16H 50/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Roche Diabetes Care GmbH
68305 Mannheim (DE)**

(72) Inventors:
• **KLOPFENSTEIN, Yannick**
**8048 Zurich (CH)**
• **LUSTENBERGER, Patrick**
**8048 Zurich (CH)**
• **RINGEMANN, Christian**
**68305 Mannheim (DE)**

(74) Representative: **Altmann Stößel Dick
Patentanwälte PartG mbB
Theodor-Heuss-Anlage 2
68165 Mannheim (DE)**

(54) **SYSTEM AND METHOD FOR BLOOD GLUCOSE PREDICTION**

(57) A blood glucose management system is disclosed that incorporates a blood glucose prediction model configured to predict future blood glucose measurements of a person, such as a person with diabetes, on the basis of historical blood glucose measurements, insulin bolus dosage data, and carbohydrate intake data. The historical data are pre-processed to obtain a set of features that are advantageous for predicting future blood glucose measurements using the blood glucose prediction model. The predicted future blood glucose measurements enable the blood glucose management system to provide easy-to-understand visualizations of health information. The predicted future blood glucose measurements also enable the blood glucose management system to provide early warnings of a hypoglycemic condition or hyperglycemic condition of the person.

FIG. 1

EP 4 610 994 A1

EP 4 610 994 A1



**Description**

FIELD

The system and method disclosed in this document relate to blood glucose management and, more particularly, to predicting future blood glucose values of a person based on historical data.

BACKGROUND

Unless otherwise indicated herein, the materials described in this section are not admitted to be the prior art by inclusion in this section.

In many fields of medical treatment and healthcare, the monitoring of certain body functions is required. For people with diabetes, a regular check of their blood glucose concentration level is typically part of the person's daily routine. Preferably, the blood glucose concentration level is measured at least several times per day, so that the person can determine when to initiate a responsive medication (such as insulin or an insulin analog) when certain limits are exceeded. In order not to unduly disrupt the daily routine of the person, in many cases a portable medical test device is used. Many different types of portable medical test devices for monitoring various body functions, including glucose concentration levels, are commercially available.

One type of medical test device is a continuous glucose monitor ("CGM"). The typical CGM includes a body worn sensor that communicates electronically with a computing device, such as a smartphone, that runs a corresponding application or "app." A benefit of CGMs is that the person's blood glucose concentration level is monitored periodically, for example, a new glucose concentration level reading may be generated every five minutes. Another benefit of CGMs is that users do not have to prick their finger to draw blood for testing throughout the day. As a result, CGMs have become popular with people with diabetes.

Often the smartphone in communication with the CGM is configured to generate alerts and/or alarms based on the user's blood glucose concentration level data. For example, the smartphone may generate an alert when the glucose concentration level data indicates that the person is experiencing a hypoglycemic condition or hyperglycemic condition. However, such alerts and/or alarms are generally only provided when the person is already or will soon be experiencing the hypoglycemic condition or hyperglycemic condition. Additionally, if such alerts and/or alarms are provided erroneously or too frequently, they may be ignored or not taken seriously by the user.

Based on the above-described deficiencies of currently available CGMs and corresponding smartphone applications, it is desirable to provide a method for predicting future blood glucose values of a person that is sufficiently robust and accurate so that alerts and/or alarms of a predicted hypoglycemic condition or hyperglycemic condition can be provided earlier to the person, while minimizing erroneously provided alerts and/or alarms.

SUMMARY

A method for providing health information is disclosed. The method comprises receiving, with a processor, historical blood glucose measurements of a person measured prior to a cut-off time. The method further comprises determining, with the processor, an input tensor comprising a plurality of feature vectors based on the historical blood glucose measurements, each respective feature vector corresponding to a respective time segment from a first time window that ends prior to the cut-off time. The method further comprises determining, with the processor, based on the input tensor using a machine learning model, predicted blood glucose measurements of the person for a second window of time that starts after the cut-off time. The method further comprises providing at least one of (i) a visualization of the predicted future blood glucose measurements on a display of an electronic device and (ii) a perceptible warning of a possible future health status via the electronic device.

In one embodiment, the determining the input tensor further comprises determining, for each respective time segment from the first time window, the respective feature vector by determining a respective value for each feature of a plurality of features.

In one embodiment, the determining the input tensor further comprises calculating, for each respective time segment from the first time window, as one of the plurality of features of the respective feature vector, an average blood glucose measurement of the person calculated over a predetermined duration of time ending at an end of the respective time segment. In one embodiment, the determining the input tensor further comprises calculating, for each respective time segment from the first time window, as features of the plurality of features of the respective feature vector, a plurality of average blood glucose measurements of the person, each average blood glucose measurement being calculated over a different predetermined duration of time ending at the end of the respective time segment.

In one embodiment, the determining the input tensor further comprises determining, for each respective time segment from the first time window, as one of the plurality of features of the respective feature vector, an average blood glucose measurement of the person calculated over a plurality of previous time segments of a plurality of previous days that are a same time of day as the respective time segment.

In one embodiment, the determining the input tensor further comprises determining, for each respective time segment from the first time window, as one of the plurality of features of the respective feature vector, an average blood glucose measurement of the person calculated over a plurality of previous time segments of a plurality of previous days that are a same time of day as the respective time segment, shifted forward in time by a predetermined amount of time.

In one embodiment, the method further comprises receiving, with the processor, insulin intake data indicating insulin doses taken by the person prior to the cut-off time. The input tensor is determined based on the historical blood glucose measurements and the insulin intake data.

In one embodiment, the determining the input tensor further comprises calculating, for each respective time segment from the first time window, as one of the plurality of features of the respective feature vector, using an insulin absorption model, an estimated insulin on board of the person at a respective time that is a predetermined amount of time after an end of the respective time segment, the respective time being subsequent to the cut-off time.

In one embodiment, the method further comprises receiving, with the processor, carbohydrate consumption data indicating carbohydrates consumed by the person prior to the cut-off time. The input tensor is determined based on the historical blood glucose measurements and the carbohydrate consumption data.

In one embodiment, the determining the input tensor further comprises calculating, for each respective time segment from the first time window, as one of the plurality of features of the respective feature vector, using a carbohydrate absorption model, an estimated amount of unabsorbed carbohydrates in the person at a respective time that is a predetermined amount of time after an end of the respective time segment, the respective time being subsequent to the cut-off time.

In one embodiment, the determining the input tensor further comprises calculating, for each respective time segment from the first time window, a respective normalized time of day based on a time of day of an end of the respective time segment, the respective normalized time of day being a value between zero and two-pi radians. The determining the input tensor further comprises calculating, as at least one of the plurality of features of the respective feature vector, at least one of (i) a cosine of the normalized time of day and (ii) a sine of the normalized time of day.

In one embodiment, the machine learning model includes a recurrent neural network having an encoder-decoder architecture. An encoder of the recurrent neural network having a first plurality of gated recurrent unit cells and a decoder of the recurrent neural network having a second plurality of gated recurrent unit cells.

In one embodiment, the determining the predicted blood glucose measurements further comprises determining a scaled input tensor by scaling values of each respective feature in the plurality of feature vectors to fall within a respective predetermined range for the respective feature. The determining the predicted blood glucose measurements further comprises determining the predicted blood glucose measurements based on the scaled input tensor using the machine learning model.

In one embodiment, the determining the predicted blood glucose measurements further comprises determining an output tensor using the machine learning model. The determining the predicted blood glucose measurements further comprises mapping the output tensor to an output vector including the predicted blood glucose measurements.

In one embodiment, the determining the predicted blood glucose measurements further comprises determining a scaled output vector including scaled predicted blood glucose measurements by scaling the output vector such that the scaled predicted blood glucose measurements fall between a minimum blood glucose measurement and a maximum blood glucose measurement.

In one embodiment, the determining the predicted blood glucose measurements further comprises determining a calibrated output vector including calibrated predicted blood glucose measurements by adding a bias value to each of the predicted blood glucose measurements.

In one embodiment, the determining the predicted blood glucose measurements further comprises determining, for each predicted blood glucose measurements, a respective upper error bound and a respective lower error bound.

In one embodiment, the providing the visualization further comprises displaying, on the display of the electronic device, a visualization of each of the predicted future blood glucose measurements in association with the respective upper error bound and the respective lower error bound.

In one embodiment, the providing the perceptible warning further comprises determining that the predicted future blood glucose measurements indicate one of (i) a possible hyperglycemic condition of the person and (ii) a possible hypoglycemic condition of the person. The providing the perceptible warning further comprises outputting, via an output device of the electronic device, the perceptible warning in response to determining that the predicted future blood glucose measurements indicate the one of (i) the possible hyperglycemic condition of the person and (ii) the possible hypoglycemic condition of the person.

The method may be computer implemented. The term "computer implemented" as used herein is a broad term and is to be given its ordinary and customary meaning to a person of ordinary skill in the art and is not to be limited to a special or customized meaning. The term specifically may refer, without limitation, to a method involving at least one computer and/or at least one computer network. The computer and/or computer network may comprise at least one processor which is configured for performing at least one of the method steps of the method according to the present invention. Preferably each of the method steps is performed by the computer and/or computer network. The method may be performed completely automatically, specifically without user interaction.

A system for providing health information is also disclosed. The system comprises a memory device. The system further comprises at least one output device. The system further comprises a processor operably connected to the memory device and the least one output device. The processor is configured to receive, and store in the memory device, historical blood glucose measurements of a person measured prior to a cut-off time. The processor is further configured to determine an input tensor comprising a plurality of feature vectors based on the historical blood glucose measurements, each respective feature vector corresponding to a respective time segment from a first time window that ends prior to the cut-off time. The processor is further configured to determine, based on the input tensor using a machine learning model, predicted blood glucose measurements of the person for a second window of time that starts after the cut-off time. The processor is further configured to operate the at least one output device to at least one of (i) display a visualization of the predicted future blood glucose measurements and (ii) output a perceptible warning of a possible future health status.

Further disclosed and proposed herein is a computer program including computer-executable instructions for performing the method according to the present invention in one or more of the embodiments enclosed herein when the instructions are executed on a computer or computer network. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps as indicated above may be performed by using a computer or a computer network, preferably by using a computer program.

Further disclosed and proposed herein is a computer program product having program code means, in order to perform the method according to the present invention in one or more of the embodiments enclosed herein when the program is executed on a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the method according to one or more of the embodiments disclosed herein.

Further disclosed and proposed herein is a non-transient computer-readable medium including instructions that, when executed by one or more processors, cause the one or more processors to perform the method according to one or more of the embodiments disclosed herein.

Further disclosed and proposed herein is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Finally, disclosed and proposed herein is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:

- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

Summarizing and without excluding further possible embodiments, the following embodiments may be envisaged:

Embodiment 1. A method for providing health information, the method comprising:

receiving, with a processor, historical blood glucose measurements of a person measured prior to a cut-off time;
determining, with the processor, an input tensor comprising a plurality of feature vectors based on the historical blood glucose measurements, each respective feature vector corresponding to a respective time segment from a first time window that ends prior to the cut-off time;
determining, with the processor, based on the input tensor using a machine learning model, predicted blood glucose measurements of the person for a second window of time that starts after the cut-off time; and
providing at least one of (i) a visualization of the predicted future blood glucose measurements on a display of an electronic device and (ii) a perceptible warning of a possible future health status via the electronic device.

Embodiment 2. The method according to embodiment 1, the determining the input tensor further comprising: determining, for each respective time segment from the first time window, the respective feature vector by determining a respective value for each feature of a plurality of features.

Embodiment 3. The method according to embodiment 2, the determining the input tensor further comprising: calculating, for each respective time segment from the first time window, as one of the plurality of features of the respective feature vector, an average blood glucose measurement of the person calculated over a predetermined duration of time ending at an end of the respective time segment.

Embodiment 4. The method according to embodiment 3, the determining the input tensor further comprising: calculating, for each respective time segment from the first time window, as features of the plurality of features of the respective feature vector, a plurality of average blood glucose measurements of the person, each average blood glucose measurement being calculated over a different predetermined duration of time ending at the end of the respective time segment.

Embodiment 5. The method according to embodiment 2, the determining the input tensor further comprising: determining, for each respective time segment from the first time window, as one of the plurality of features of the respective feature vector, an average blood glucose measurement of the person calculated over a plurality of previous time segments of a plurality of previous days that are a same time of day as the respective time segment.

Embodiment 6. The method according to embodiment 2, the determining the input tensor further comprising: determining, for each respective time segment from the first time window, as one of the plurality of features of the respective feature vector, an average blood glucose measurement of the person calculated over a plurality of previous time segments of a plurality of previous days that are a same time of day as the respective time segment, shifted forward in time by a predetermined amount of time.

Embodiment 7. The method according to embodiment 2 further comprising:

receiving, with the processor, insulin intake data indicating insulin doses taken by the person prior to the cut-off time,
wherein the input tensor is determined based on the historical blood glucose measurements and the insulin intake data.

Embodiment 8. The method according to embodiment 7, the determining the input tensor further comprising:

calculating, for each respective time segment from the first time window, as one of the plurality of features of the respective feature vector, using an insulin absorption model, an estimated insulin on board of the person at a respective time that is a predetermined amount of time after an end of the respective time segment, the respective time being subsequent to the cut-off time.

Embodiment 9. The method according to embodiment 2 further comprising:

> receiving, with the processor, carbohydrate consumption data indicating carbohydrates consumed by the person prior to the cut-off time,
> wherein the input tensor is determined based on the historical blood glucose measurements and the carbohydrate consumption data.

Embodiment 10. The method according to embodiment 9, the determining the input tensor further comprising: calculating, for each respective time segment from the first time window, as one of the plurality of features of the respective feature vector, using a carbohydrate absorption model, an estimated amount of unabsorbed carbohydrates in the person at a respective time that is a predetermined amount of time after an end of the respective time segment, the respective time being subsequent to the cut-off time.

Embodiment 11. The method according to embodiment 2, the determining the input tensor further comprising:

> calculating, for each respective time segment from the first time window, a respective normalized time of day based on a time of day of an end of the respective time segment, the respective normalized time of day being a value between zero and two-pi radians; and
> calculating, as at least one of the plurality of features of the respective feature vector, at least one of (i) a cosine of the normalized time of day and (ii) a sine of the normalized time of day.

Embodiment 12. The method according to embodiment 1, wherein the machine learning model includes a recurrent neural network having an encoder-decoder architecture, an encoder of the recurrent neural network having a first plurality of gated recurrent unit cells and a decoder of the recurrent neural network having a second plurality of gated recurrent unit cells.

Embodiment 13. The method according to embodiment 1, the determining the predicted blood glucose measurements further comprising:

> determining a scaled input tensor by scaling values of each respective feature in the plurality of feature vectors to fall within a respective predetermined range for the respective feature; and
> determining the predicted blood glucose measurements based on the scaled input tensor using the machine learning model.

Embodiment 14. The method according to embodiment 1, the determining the predicted blood glucose measurements further comprising:

> determining an output tensor using the machine learning model; and
> mapping the output tensor to an output vector including the predicted blood glucose measurements.

Embodiment 15. The method according to embodiment 14, the determining the predicted blood glucose measurements further comprising:
determining a scaled output vector including scaled predicted blood glucose measurements by scaling the output vector such that the scaled predicted blood glucose measurements fall between a minimum blood glucose measurement and a maximum blood glucose measurement.

Embodiment 16. The method according to embodiment 14, the determining the predicted blood glucose measurements further comprising:
determining a calibrated output vector including calibrated predicted blood glucose measurements by adding a bias value to each of the predicted blood glucose measurements.

Embodiment 17. The method according to embodiment 1, the determining the predicted blood glucose measurements further comprising:

determining, for each predicted blood glucose measurements, a respective upper error bound and a respective lower error bound.

Embodiment 18. The method according to embodiment 17, the providing the visualization further comprising: displaying, on the display of the electronic device, a visualization of each of the predicted future blood glucose measurements in association with the respective upper error bound and the respective lower error bound.

Embodiment 19. The method according to embodiment 1, the providing the perceptible warning further comprising:

determining that the predicted future blood glucose measurements indicate one of (i) a possible hyperglycemic condition of the person and (ii) a possible hypoglycemic condition of the person; and outputting, via an output device of the electronic device, the perceptible warning in response to determining that the predicted future blood glucose measurements indicate the one of (i) the possible hyperglycemic condition of the person and (ii) the possible hypoglycemic condition of the person.

Embodiment 20. The method according to any one of the preceding embodiments, wherein the method is computer implemented.

Embodiment 21. A system for providing health information, the system comprising:

a memory device;
at least one output device;
a processor operably connected to the memory device and the least one output device, the processor being configured to:

receive, and store in the memory device, historical blood glucose measurements of a person measured prior to a cut-off time;
determine an input tensor comprising a plurality of feature vectors based on the historical blood glucose measurements, each respective feature vector corresponding to a respective time segment from a first time window that ends prior to the cut-off time;
determine, based on the input tensor using a machine learning model, predicted blood glucose measurements of the person for a second window of time that starts after the cut-off time; and
operate the at least one output device to at least one of (i) display a visualization of the predicted future blood glucose measurements and (ii) output a perceptible warning of a possible future health status.

Embodiment 22. A computer program comprising instructions which, when the program is executed by the system according to the preceding embodiment, cause the system to perform the method according to any one of the preceding embodiments referring to a method.

Embodiment 23. A computer-readable storage medium comprising instructions which, when the instructions are executed by the system according to embodiment 21, cause the system to perform the method according to any one of the preceding embodiments referring to a method.

Embodiment 24. A non-transient computer-readable medium including instructions that, when executed by one or more processors, cause the one or more processors to perform the method according to any one of the preceding embodiments referring to a method.

BRIEF DESCRIPTION OF THE DRAWINGS

The foregoing aspects and other features of the system and method are explained in the following description, taken in connection with the accompanying drawings.

FIG. 1 summarizes a blood glucose prediction functionality of a blood glucose management system.
FIG. 2 shows an exemplary blood glucose management system, which is typically used by a person with diabetes.
FIG. 3 shows a data plot summarizing the inputs and outputs of the blood glucose prediction model.
FIG. 4 shows a logical flow diagram illustrating the operations of an exemplary blood glucose prediction model.
FIG. 5 shows a flow diagram for a method for providing health information.
FIG. 6 shows a portion of a graphical user interface including data plot 600 that visualizes the predicted future blood glucose measurements.

FIG. 7 shows a portion of a graphical user interface including visual alert that lets a user know that his or her blood glucose has a high probability of falling below a threshold value.

## DETAILED DESCRIPTION

For the purposes of promoting an understanding of the principles of the disclosure, reference will now be made to the embodiments illustrated in the drawings and described in the following written specification. It is understood that no limitation to the scope of the disclosure is thereby intended. It is further understood that the present disclosure includes any alterations and modifications to the illustrated embodiments and includes further applications of the principles of the disclosure as would normally occur to one skilled in the art to which this disclosure pertains.

### Overview

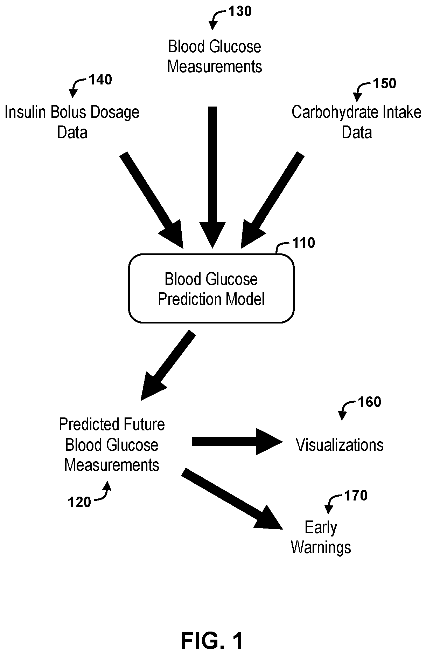

FIG. 1 summarizes a blood glucose prediction functionality of a blood glucose management system. The blood glucose management system advantageously incorporates a blood glucose prediction model 110 configured to predict future blood glucose measurements 120, with uncertainty estimates, of a person such as a person with diabetes, on the basis of historical blood glucose measurements 130, insulin bolus dosage data 140, and carbohydrate intake data 150. By providing users with real-time glucose predictions and their corresponding uncertainty estimates, the blood glucose management system advantageously to empowers users to make informed decisions and take proactive measures to effectively manage their glycemic control.

The historical blood glucose measurements 130, the insulin bolus dosage data 140, and the carbohydrate intake data 150 are pre-processed to obtain a set of features that are advantageous for predicting future blood glucose measurements using the blood glucose prediction model 110. In at least some embodiments, the blood glucose prediction model 110 advantageously adopts a is a Recurrent Neural Network (RNN) having an Encoder-Decoder (or Sequence-to-Sequence) architecture for processing the extracted features to determine the predicted future blood glucose measurements 120.

Additionally, the predicted future blood glucose measurements 120 advantageously enable the blood glucose management system to provide easy-to-understand visualizations 160 of health information, including the historical blood glucose measurements 130 and the predicted future blood glucose measurements 120. In this way, the person can more easily and better understand their health and how to better manage their blood glucose concentration throughout the day. The predicted future blood glucose measurements 120 can help the person to better appreciate the relationships that time of day, insulin bolus dosing, and the carbohydrate intake can have on his or her blood glucose concentration.

The predicted future blood glucose measurements 120 also advantageously enable the blood glucose management system to provide early warnings 170 of a hypoglycemic condition or hyperglycemic condition of the person. Such early warnings 170 are helpful for a person, such as a person with diabetes, who must carefully manage his or her blood glucose concentration.

### Exemplary Blood Glucose Management System

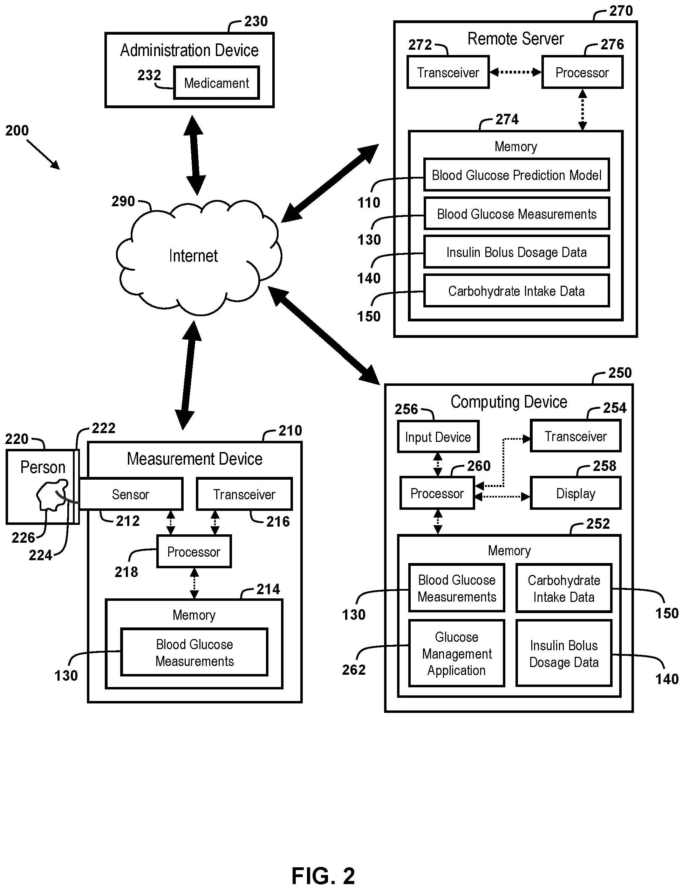

FIG. 2 shows an exemplary blood glucose management system 200, which is typically used by a person with diabetes. The blood glucose management system 200 includes a measurement device 210, an administration device 230, a computing device 250, and a remote server 270. It should be appreciated, however, that the components of the blood glucose management system 200 shown and described are merely exemplary and that the blood glucose management system 200 may comprise any alternative configuration.

The measurement device 210 of the blood glucose management system 200 is a body-worn continuous glucose monitor ("CGM") that is used to generate the blood glucose measurements 130 that correspond to a person's blood glucose concentration. The measurement device 210 includes a sensor 212, a memory device 214, and a transceiver 216, each operably connected to a processor 218.

The sensor 212 of the measurement device 210 is mounted on the skin 222 of a person 220 with an adhesive and includes a probe 224 that is positioned just under the skin 222. The probe 224 is in contact with interstitial fluid 226 of the person 220. In one embodiment, the probe 224 is an enzyme-based amperometric biosensor that is configured to measure blood glucose measurements 130 in the interstitial fluid 226. In other embodiments, the sensor 212 measures glucose concentrations according to other suitable structural configurations and methodologies. The measurement device 210 may operate with or without a corresponding insulin pump (shown as an embodiment of the administration device 230, for example).

The processor 218 of the measurement device 210 is configured to execute instructions to operate the measurement device 210 to enable the features, functionality, characteristics, and/or the like as described herein.

The processor 218 generally comprises one or more processors which may operate in parallel or otherwise in concert with one another. It will be recognized by those of ordinary skill in the art that the term "processor" as used herein includes any hardware system, hardware mechanism, or hardware component that processes data, signals, or other information. Accordingly, the processor 218 may include a system with a central processing unit, graphics processing units, multiple processing units, dedicated circuitry for achieving functionality, programmable logic, or other processing systems.

The memory device 214 of the measurement device 210 is configured to store data and program instructions that, when executed by the processor 218, enable the measurement device 210 to perform various operations described herein. The memory device 214 may be any type of electronic device capable of storing information accessible by the processor 218, such as a memory card, read only memory ("ROM"), random access memory ("RAM"), a hard drive, a solid state drive, a disc, flash memory, or any of various other computer-readable media serving as data storage devices, as will be recognized by those of ordinary skill in the art. The memory device 214 is also referred to herein as a non-transitory computer readable medium, a non-transitory memory device, and a non-transitory memory. The memory device 214 stores the blood glucose measurements 130 generated by the processor 218 and as measured by the sensor 212.

The transceiver 216 of the measurement device 210, in one embodiment, is configured for the wired and/or wireless exchange of data with the computing device 250. The transceiver 216 includes one or more modems, processors, memories, oscillators, antennas, or other hardware conventionally included in a communications module to enable electronic communications with various other devices. For example, the transceiver 216 may exchange electronic data using a wireless local area network ("Wi-Fi"), a personal area network, Bluetooth®, near-field communication ("NFC"), ultra-wide band ("UWB"), a cellular network, and/or any other wireless network protocol. Accordingly, the transceiver 216 is compatible with any desired wireless communication standard or protocol including, but not limited to, IEEE 802.11, IEEE 802.15.1 ("Bluetooth®"), Global System for Mobiles ("GSM"), and Code Division Multiple Access ("CDMA"). In one embodiment, the transceiver 216 operably connects the measurement device 210 to the Internet 290 for data exchange with any other Internet 290 connected device. In another embodiment, the transceiver 216 transmits and receives data directly to the computing device 250 without being connected to the Internet 290. The transceiver 216 is also referred to herein as a network adapter, a network device, and/or a network communication module.

With continued reference to FIG. 2, the administration device 230 of the blood glucose management system 200 is a medicament delivery device. The administration device 230 is operably connected to the measurement device 210 and the computing device 250 to receive insulin bolus administration data, for example. In some embodiments, the administration device 230 is also operably connected to the Internet 290 via a corresponding transceiver (not shown). Thus, the administration device 230 is operably connected to other Internet-connected devices, such as the remote server 270 and/or a computer (not shown) used by a doctor or a pharmacist. The administration device 230 is connected or is connectable to the person 220 to deliver the medicament 232.

In an exemplary embodiment, the administration device 230 is a smart insulin pump, and the medicament 232 is insulin, a rapid-acting insulin analog, or a regular insulin analog, each of which is referred to interchangeably herein as "insulin." The insulin pump delivers the medicament 232 to the person 220 through a thin tube (cannula, not shown) that goes under the person's skin. In another embodiment, the administration device 230 is a smart insulin "pen." The smart insulin pen delivers the medicament 232 to the person 220 through a reusable injection device (i.e., a needle).

The administration device 230 is electronically configurable to deliver a predetermined dosage (i.e., a bolus dose) of the medicament 232 to the person 220 based on the insulin bolus administration data received from the computing device 250, for example. The medicament 232 is capable of reducing the blood glucose concentration level of the person 220, and, therefore, is provided to the person 220 when a hyperglycemic condition is present or is predicted in order to assist the person 220 in returning their glucose concentration level to a lower desired level.

With continued reference to FIG. 2, the computing device 250 of the blood glucose management system 200 includes a memory device 252, a transceiver 254, an input device 256, and a display screen 258 each operably connected to a processor 260. The computing device 250 is described and illustrated herein as a smartphone. It will be appreciated that the illustrated embodiment of the computing device 250 is only one exemplary embodiment and is merely representative of any of various manners, configurations, or combinations of a personal computer, a desktop computer, a laptop computer, a smartwatch, a mobile phone, a tablet computer, or any other computing device that is operative in the manner set forth herein.

The processor 260 of the computing device 250 is configured to execute instructions to operate the computing device 250 to enable the features, functionality, characteristics, and/or the like as described herein. The processor 260 generally comprises one or more processors which may operate in parallel or otherwise in concert with one another. The processor 260 may include a system with a central processing unit, graphics processing units, multiple processing units, dedicated circuitry for achieving functionality, programmable logic, or other processing systems. The processor 260 is configured to run applications (i.e., "apps"), at least including a glucose management application 262.

The memory device 252 of the computing device 250 is configured to store data and program instructions that, when executed by the processor 260, enable the computing device 250 to perform various operations and methods described herein. The memory device 252 may be any type of electronic device capable of storing information accessible by the processor 260, such as a memory card, ROM, RAM, a hard drive, a solid state drive, a disc, flash memory, or any of various other computer-readable media serving as data storage devices, as will be recognized by those of ordinary skill in the art. The memory device 252 is also referred to herein as a non-transitory computer readable medium, a non-transitory memory device, and a non-transitory memory. The memory device 252 is configured to the historical blood glucose measurements 130, the insulin bolus dosage data 140, and the carbohydrate intake data 150.

The transceiver 254 of the computing device 250 is configured for the wired and/or wireless exchange of data with the measurement device 210, the administration device 230, the remote server 270, and the Internet 290. The transceiver 254 includes one or more modems, processors, memories, oscillators, antennas, or other hardware conventionally included in a communications module to enable electronic communications with various other devices. For example, the transceiver 254 may exchange data using Wi-Fi, a personal area network, Bluetooth®, NFC, UWB, a cellular network, and/or any other wireless network protocol. Accordingly, the transceiver 254 is compatible with any desired wireless communication standard or protocol including, but not limited to, IEEE 802.11, Bluetooth®", GSM, and CDMA. The transceiver 254 operably connects the computing device 250 to the Internet 290 for data exchange with any other Internet 290 connected device. Additionally, the transceiver 254 transmits and receives data from the measurement device 210 either directly or indirectly. The transceiver 254 is also referred to herein as a network adapter and/or a network device.

The display screen 258 of the computing device 250 is configured to render and to display a graphical user interface including text, images, and other user sensible outputs and visually comprehensible data including, but not limited to, a visualization of the historical blood glucose measurements 130 and a visualization of the predicted future blood glucose measurements 120. Additionally, graphical user interfaces of the display screen 258 may include one or more notifications or alerts relating to a current or predicted health status, such as a hypoglycemic condition or hyperglycemic condition, of the person 220. The display screen 258 may comprise any of various known types of displays, such as liquid crystal displays ("LCD") or organic light emitting diode ("OLED") screens.

In at least some embodiments, the input device 256 of the computing device 250 includes a touchscreen applied over the display screen 258 that is configured to generate user input data in response to the touch of a finger or a stylus. The input device 256 may also include at least one button, switch, keyboard, and/or keypad that is configured to generate user input data when touched or moved by a user. Additionally, or alternatively, the input device 256 includes a microphone configured to generate user input data in response to sounds, such as the voice of a user of the computing device 250. In yet another embodiment, the input device 256 is any device configured to generate user input data, as recognized by those of ordinary skill in the art.

With continued reference to FIG. 2, the remote server 270 of the blood glucose management system 200 includes a transceiver 272 and a memory device 274 operably connected to a processor 276.

The processor 276 is configured to execute instructions to operate the remote server 270 to enable the features, functionality, characteristics and/or the like as described herein. The processor 276 generally comprises one or more processors which may operate in parallel or otherwise in concert with one another. The processor 276 may include a system with a central processing unit, graphics processing units, multiple processing units, dedicated circuitry for achieving functionality, programmable logic, or other processing systems.

The transceiver 272, in one embodiment, is configured for the wired and/or wireless exchange of data with the computing device 250 and the Internet 290. The transceiver 272 includes one or more modems, processors, memories, oscillators, antennas, or other hardware conventionally included in a communications module to enable electronic communications with various other devices. For example, the transceiver 272 may exchange data using Wi-Fi, a personal area network, Bluetooth®, NFC, UWB, a cellular network, and/or any other wireless network protocol. Accordingly, the transceiver 272 is compatible with any desired wireless communication standard or protocol including, but not limited to IEEE 802.11, Bluetooth®, GSM, and CDMA. The transceiver 272 is also referred to herein as a network adapter and/or a network device.

The memory device 274 is configured to store data and program instructions that, when executed by the processor 276, enable the remote server 270 to perform various operations and methods described herein. The memory device 274 may be of any type of electronic device capable of storing information accessible by the processor 276, such as a memory card, ROM, RAM, hard drives, solid state drives, discs, flash memory, or any of various other computer-readable medium serving as data storage devices, as will be recognized by those of ordinary skill in the art. The memory device 274 is also referred to herein as a non-transitory computer readable medium, a non-transitory memory device, and a non-transitory memory. The memory device 274 at least stores the blood glucose prediction model 110. Additionally, in at least some embodiments, the memory device 274 stores the historical blood glucose measurements 130, the insulin bolus dosage data 140, and the carbohydrate intake data 150.

As illustrated in FIG. 2, the blood glucose prediction model 110 is stored in the memory device 274 of the remote server 270 and is likewise executed by the processor 276 based on data received from the measurement device 110 and from the computing device 250. In other words, the blood glucose prediction model 110 is operated "in the cloud." However, it should be appreciated that in alternative embodiments, the blood glucose prediction model 110 may be stored locally on the memory device 252 of the computing device 250 (e.g., as a part of the glucose management application 262) and executed locally by the processor 260 of the computing device 250.

Inputs and Outputs of the Blood Glucose Prediction Model

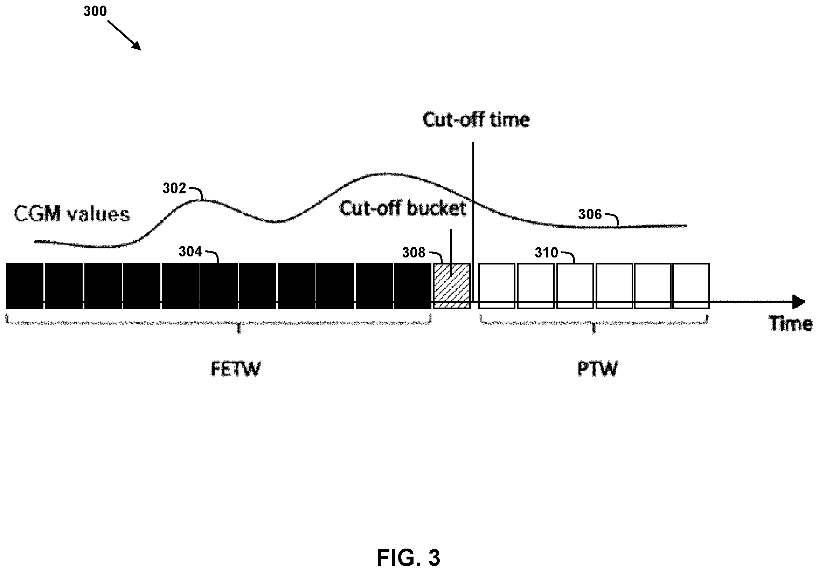

FIG. 3 shows a data plot 300 summarizing the inputs and outputs of the blood glucose prediction model 110. Particularly, during a feature extraction time window (FETW), continuous glucose monitoring values 302 (i.e., a subset of the historical blood glucose measurements 130), with respect to the person 220. In at least some embodiments, the insulin bolus dosage data 140, and the carbohydrate intake data 150 are also collected during the feature extraction time window. The feature extraction time window has a first predetermined duration (e.g., 4 hours) and is divided into a plurality of time segments 304 having a second predetermined duration (e.g., 5 minutes).

The historical blood glucose measurements 130 take the form of a time series of blood glucose concentration measurements, for example having the units 'mg/dL' (milligrams per deciliter). Each blood glucose concentration measurement is associated with a respective timestamp indicating a time at which the value was measured. For example, one blood glucose measurement might indicate that the person 220 had a blood glucose concentration of 162 mg/dL at 7:05 AM and, later, a blood glucose concentration of 165 mg/dL at 7:10 AM.

Additionally, the insulin bolus dosage data 140 take the form of a time series of insulin bolus dosage amounts (e.g., of the medicament 232), as previously received by the person 220, with associated timestamps. The insulin bolus dosage data 140 tracks both correction bolus and meal bolus received by the person 220. For example, the insulin bolus dosage data 140 might indicate that the person 220 received a meal bolus of two units of the medicament 232 at 6:00 PM, and received a correction bolus of one unit of the medicament 232 at 8:00 PM.

Similarly, the carbohydrate intake data 150 take the form of a time series of amounts of carbohydrates ("carbs") consumed by the person 220 in each meal and snack, with associated timestamps. In at least some embodiments, each amount of carbohydrates in the carbohydrate intake data 150 is classified or otherwise labeled with a particular carbohydrate type. Exemplary carbohydrate types may include simple carbohydrates, complex carbohydrates, and other carbohydrates. It will be appreciated that such carbohydrate type classifications generally reflect different rates at which the consumed carbohydrates are absorbed by the human body. For example, the carbohydrate intake data 150 might indicate that the person 220 consumed 35 grams of complex carbohydrates during a meal at 6:00 PM, and consumed 5 grams of simple carbohydrates during a snack at 9:00 PM.

In at least some embodiments, the sensor 212 of the measurement device 110 measures the continuous glucose monitoring values 302, with respect to the person 220. Additionally, in some embodiments, the person 220 inputs the insulin bolus dosage data 140 and the carbohydrate intake data 150 via the input device 256, for example by interacting with a graphical user interface of the glucose management application 262 on the display 258 via the input device 256. However, in other embodiments, the insulin bolus dosage data 140 and the carbohydrate intake data 150 may be determined or measured automatically.

The blood glucose measurements 130, the insulin bolus dosage data 140, and the carbohydrate intake data 150 are captured until a cut-off time, at which a process for blood glucose prediction is triggered. Based on the blood glucose measurements 130, the insulin bolus dosage data 140, and the carbohydrate intake data 150, the processor 276 executes the blood glucose prediction model 110 to determine predicted future continuous glucose monitoring values 306 (i.e., the predicted future blood glucose measurements 120) for a prediction time window (PTW). The prediction time window has a third predetermined duration (e.g., 2 hours) and is similarly divided into a plurality of segments 310 or aggregation buckets having the second predetermined duration (e.g., 5 minutes). In one example, the plurality of segments 310 or aggregation buckets are defined as:

$$B_i = [-240 \min + i * 5\, min < t \le -240\, min + i * 5\, min + 5] \text{ for } i \text{ in } [0, 1, ..., 47].$$

In at least some embodiments, values from the blood glucose measurements 130, the insulin bolus dosage data 140, and the carbohydrate intake data 150 that were collected with respect to a segment of time immediately prior to the cut-off time, referred to herein as the cut-off bucket 308, are ignored by the blood glucose prediction model 110 in determining the predicted future continuous glucose monitoring values 306.

Prior to being input to the blood glucose prediction model 110, the blood glucose measurements 130, the insulin bolus dosage data 140, and the carbohydrate intake data 150 are pre-processed by the remote server 270 or by the computing device 250. In some embodiments, for the blood glucose measurements 130, the remote server 270 or by the computing device 250 averages values lying in each time segment 304 (aggregation bucket), such that only one value is provided for each time segment 304. For the insulin bolus dosage data 140, the remote server 270 or by the computing device 250 sums values lying in each time segment 304, such that only one value is provided for each time segment 304. For carbohydrates of the same type in the carbohydrate intake data 150, the remote server 270 or by the computing device 250 sums values lying in each time segment 304, such that only one value is provided for each time segment 304 for each

carbohydrate type. For carbohydrates of different types in the carbohydrate intake data 150, the remote server 270 or by the computing device 250 concatenates values lying in each time segment 304.

Additionally, the processor 272 of the remote server 270 and/or the processor 260 of the computing device 250 determines an input tensor $X$ having a plurality of feature vectors $X_i$ corresponding to a sequence of respective time segments $i$ (e.g., the time segments 304 of the feature extraction time window) of predetermined length (e.g., 5 minutes). Each feature vector $X_i$ comprises values for a plurality of features for the respective time segment $i$. Based on the feature vector $X$, the processor 276 executes the blood glucose prediction model 110 to determine an output tensor, which can be mapped to an output vector $Y$ that represents the predicted future blood glucose measurements 120 within the prediction time window.

In the embodiment described in detail herein, a first dimension of the input tensor $X$ includes each sample provided to the blood glucose prediction model 110. When the blood glucose prediction model 110 is deployed, this first dimension will always be one. However, during training, the blood glucose prediction model 110 can accept multiple samples to enable the training of the model in the development phase. A second dimension of the input tensor $X$ includes the plurality of feature vectors $X_i$. Particularly, in at least one embodiment, the input tensor $X$ includes 48 feature vectors $X_i$, denoted $X_{47}$-$X_0$, corresponding to 48 sequential time segments of the feature extraction time window. Each feature vector aggregates data from a 5-minute time segment, such that the input tensor $X$ aggregates features extracted from the prior 240 minutes. In at least one embodiment, the 48 feature vectors $X_{47}$-$X_0$ are organized within the input tensor $X$ from oldest to most recent. Finally, the third dimension of the input tensor $X$ includes 8 different features that summarize the input data. It should be appreciated, however, that the input tensor $X$ may include any number of dimensions that represent any number of features from any number of different time intervals.

The plurality of features in each feature vector $X_i$ include one or multiple different average blood glucose measurements of the person 220. Each different average blood glucose measurement is calculated over a different predetermined duration of time ending at an end of the respective time segment $i$.

In one embodiment, the processor 272 and/or the processor 260 determines, as a first feature of each feature vector $X_i$, referred to herein as the feature "CGM," an average blood glucose measurement calculated between the times $T$ - 5 *minutes* and $T$, where $T$ denotes the time at which the respective time segment $i$ ends.

In one embodiment, the processor 272 and/or the processor 260 determines, as a second feature of each feature vector $X_i$, referred to herein as the feature "CGM_AVG_6H," an average blood glucose measurement calculated between the times $T$ - 6 *hours* and $T$, where $T$ denotes the time at which the respective time segment $i$ ends.

In one embodiment, the processor 272 and/or the processor 260 determines, as a third feature of each feature vector $X_i$, referred to herein as the feature "CGM AVG 1D," an average blood glucose measurement calculated between the times $T$ - 1 *day* and $T$, where $T$ denotes the time at which the respective time segment $i$ ends.

In one embodiment, the processor 272 and/or the processor 260 determines, as a fourth feature of each feature vector $X_i$, referred to herein as the feature "CGM_AGP_14D_Q50_SHIFT_POS_264," a 50% quantile value of an ambulatory glucose profile of the person over a period of time. The ambulatory glucose profile is calculated from the last fourteen days of historical blood glucose measurements 120, not including the current day, but shifted forward in time by a predetermined amount of time. It at least some embodiments, the predetermined amount of time is equal to a duration of the prediction time window (e.g., 2 hours). For each time segment $i$, the processor 272 and/or the processor 260 determines an average blood glucose measurement of the person during previous time segments of a plurality of previous days (e.g., 14 days) that are a same time of day as the respective time segment $i$. For example, if the respective time segment $i$ occurs between 7:00AM and 7:05 AM, then the 50% quantile value of an ambulatory glucose profile is calculated as an average blood glucose measurement between 7:00AM and 7:05 AM over the plurality of previous days (e.g., 14 days), not including the current day.

In at least some embodiments, the previous time segments of the plurality of previous days are shifted forward in time by a duration of the second time window (e.g., 2 hours into the future). In this way, the feature at each time segment represents a 50% quantile value of the ambulatory glucose profile, shifted into the future. For example, if the respective time segment $i$ occurs between 7:00AM and 7:05 AM, then the 50% quantile value of an ambulatory glucose profile is calculated as an average blood glucose measurement between 9:00AM and 9:05 AM over the plurality of previous days (e.g., 14 days), not including the current day.

In one embodiment, the processor 272 and/or the processor 260 determines, as a fifth feature of each feature vector $X_i$, referred to herein as the feature "BOLUS_INSULIN_ON_BOARD_SHIFT_NEG_24," an estimated insulin on board of the person at a respective time that is a predetermined amount of time after the time $T$, denoting the time at which the respective time segment $i$ ends. It at least some embodiments, the predetermined amount of time is equal to a duration of the prediction time window (e.g., 2 hours). In some embodiments, the processor 272 and/or the processor 260 determines the estimated insulin on board by fetching a previous time period (e.g., the last 6 hours) of bolus insulin before the cut-off time from the insulin bolus dosage data 140 and deriving the estimated insulin on board using an insulin absorption model based on the past relevant insulin injections, assuming an acting time, i.e., activity duration (e.g., 240 minutes) and an offset time, i.e., peak activity time (e.g., 60 minutes). The processor 272 and/or the processor 260 determines the estimated insulin on board by rolling over the bolus insulin time series (superposition is applied if multiple bolus injections occurred). An exemplary insulin absorption model for determining an estimated insulin on board at a time t (measured from the bolus intake) can be given according to the following formula:

$$IOB(t) = \left\{ 1 - s(1-a)\left( \left( \frac{t^2}{\tau t_d(1-a)} - \frac{t}{\tau} - 1 \right) e^{-\frac{t}{\tau}} + 1 \right) \right\} * I_0$$

where:

$\tau = \dfrac{t_p(1-\frac{t_p}{t_d})}{1-2\frac{t_p}{t_d}}$ is a time constant of exponential decay,

$a = \dfrac{2\tau}{t_d}$ is a rise time factor,

$s = \dfrac{1}{1-a+(1+a)e^{-\frac{t_d}{\tau}}}$ is an auxiliary scale factor,

$t_d$ is the activity duration (acting time),
$t_p$ is the peak activity time (offset time), and
$I_0$ is an amount of insulin injected (bolus insulin intake) at time t = 0.

In one embodiment, the processor 272 and/or the processor 260 determines, as a sixth feature of each feature vector $X_i$, referred to herein as the feature "UNABSORBED_CARBS_SHIFT_NEG_24," an estimated amount of unabsorbed carbohydrates in the person at a respective time that is a predetermined amount of time after the time $T$, denoting the time at which the respective time segment $i$ ends. It at least some embodiments, the predetermined amount of time is equal to a duration of the prediction time window (e.g., 2 hours). In some embodiments, the processor 272 and/or the processor 260 determines the estimated amount of unabsorbed carbohydrates by fetching a previous time period (e.g., the last 6 hours and 10 minutes) of carbohydrate amounts and meal type information before the cut-off time from the carbohydrate intake data 150 and deriving the estimated amount of unabsorbed carbohydrates using a carbohydrate absorption model based on the past relevant carbohydrate intakes, assuming an absorption delay (e.g., 10 minutes) and an absorption duration depending on the carbohydrate type (e.g., 120 minutes for simple, 240 minutes for complex, 180 minutes for other). The processor 272 and/or the processor 260 determines the estimated amount of unabsorbed carbohydrates by rolling over the carbohydrate intake time series (superposition is applied if multiple intakes occurred). An exemplary carbohydrate absorption model for determining the estimated amount of unabsorbed carbohydrates at a time $t$ (measured from the carbohydrate intake) can be given according to the following formula:

$$UAC(t, type) = \begin{cases} if\ t \le delay & A\dfrac{t}{delay} \\ if\ t \le d(type) & A\left( \dfrac{-1}{d(type) - delay}t + \dfrac{d(type)}{d(type) - delay} \right), \\ else & 0 \end{cases}$$

$$d(type) = \begin{cases} if\ type = simple & 120\ min \\ if\ type = complex & 240\ min, \\ else & 180\ min \end{cases}$$

where $A$ is an amount of carbohydrate intake at time $t = 0$, $delay$ is an absorption delay (e.g., 10 minutes) and $d(type)$ is an absorption duration dependent on carbohydrate type.

In one embodiment, the processor 272 and/or the processor 260 determines, as a seventh feature of each feature vector $X_i$, referred to herein as the feature "TIME_OF_DAY_UNITARY_CIRCLE_COS," a cosine of a normalized time of day. The processor 272 and/or the processor 260 determines the normalized time of day based on the time of day $T$,

denoting the time at which the respective time segment $i$ ends. The normalized time of day is a value between 0 and $2\pi$ radians. In other words, the time of day is mapped between 0 and $2\pi$ ($0:00 \rightarrow 0$, $24:00 \rightarrow 2\pi$) and then the cosine function is applied.

In one embodiment, the processor 272 and/or the processor 260 determines, as an eighth feature of each feature vector $X_i$, referred to herein as the feature "TIME_OF_DAY_UNITARY_CIRCLE_SIN," a sine of a normalized time of day. The processor 272 and/or the processor 260 determines the normalized time of day based on the time of day $T$, denoting the time at which the respective time segment $i$ ends. The normalized time of day is a value between 0 and $2\pi$ radians. In other words, the time of the day is mapped between 0 and $2\pi$ ($0:00 \rightarrow 0$, $24:00 \rightarrow 2\pi$) and then the sine function is applied.

In some embodiments, the processor 272 and/or the processor 260 forms each feature vector $X_i$ from the eight features "CGM," "CGM_AVG_6H," "CGM_AVG_1D," "CGM_AGP_14D_Q50_SHIFT_POS_264," "BOLUS_INSULIN_ON_BOARD_SHIFT _NEG_24," "UNABSORBED_CARBS_SHIFT_NEG_24," "TIME_OF_DAY_ UNITARY _CIRCLE COS," and "TIME_OFDAY_UNITARY_CIRCLE_SIN" that were calculated with respect to each time segment $i$. Finally, the processor 272 and/or the processor 260 forms the input tensor $X$ from the plurality of feature vectors $X_i$, beginning with the feature vector $X_i$ associated with the oldest time segment $i$ (e.g., $X_{47}$) and ending with the feature vector $X_i$ associated with the most recent time segment $i$ (e.g., $X_0$). The processor 272 and/or the processor 260 stores the input tensor $X$ in the memory device 252 and/or the memory device 274. In at least some embodiments, the processor 272 and/or the processor 260 also stores, alongside the input tensor $X$, the cut-off time, and a unique identifier for the person 220 with respect to which the data 130, 140, 150 was collected.

Finally, after the input tensor $X$ is determined, the processor 276 of the remote server 270 executes the blood glucose prediction model 110 to determine an output tensor, which is mapped to the output vector $Y$, which includes the predicted future blood glucose measurements 120. In at least one embodiment, the output vector $Y$ includes 24 output vectors $Y_j$, denoted $Y_{23}$- $Y_0$, corresponding to 24 sequential time segments of the prediction time window.

Model Architecture

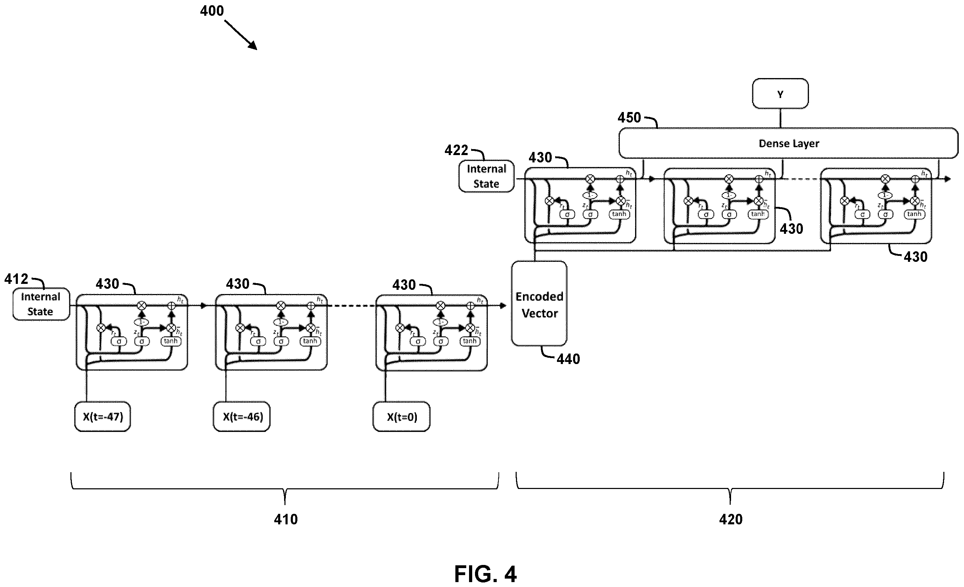

FIG. 4 shows a logical flow diagram illustrating the operations of an exemplary blood glucose prediction model 400. The blood glucose prediction model 400 is one of many possible implementations of the blood glucose prediction model 110, which may take a variety of alternative forms. However, in the illustrated embodiment, the blood glucose prediction model 400 is a Recurrent Neural Network (RNN) having an Encoder-Decoder (or Sequence-to-Sequence) architecture. The blood glucose prediction model 400 has an encoder 410 and a decoder 420. Each of the encoder 410 and decoder 420 includes a plurality of RNN cells 430. Each RNN cell in the encoder 410 and decoder 420 of the blood glucose prediction model 400 may, for example, take the form of a Gated Recurrent Unit (GRU) cell 430. However, it should be appreciated that other types of cells may be utilized, such as Long Short-Term Memory (LSTM) cells. In any case, such an architecture enables the blood glucose prediction model 110 to predict blood glucose values several timesteps into the future in one prediction step. This architecture is particularly advantageous for efficient medium-term and long-term predictions.

The blood glucose prediction model 400 receives the input tensor $X$ as its input. As discussed above, the input tensor $X$ includes a plurality of feature vectors $X_i$ (e.g., 48 feature vectors $X_i$, denoted $X_{47}$-$X_0$) corresponding to a plurality of sequential time segments of the feature extraction time window (e.g., 48 time segments). The input tensor $X$ is provided as input to the encoder 410 of the blood glucose prediction model 400. In at least one embodiment, an internal state 412 of the encoder 410 is initialized randomly. The encoder 410 transforms the input tensor $X$ into an encoded vector 440. Particularly, the input tensor $X$ is ingested by the encoder 410 step by step (oldest time point to newest). At each step the internal state 412 is updated based on the new data point and the previous state. The final encoder internal state 412 after encoding all historical data (encoded vector 440) acts as an input to the decoder 420. Subsequently, the decoder 420 transforms the encoded vector 440 into an output tensor. In at least one embodiment, an internal state 422 of the encoder 410 is initialized randomly. Step by step the output time series is created by using the encoded vector 440 (constant input over time) and the previous internal state 422 of the decoder 420 to create a new internal state 422 of the decoder 420. Finally, a dense layer 450 maps the output tensor to an output vector Y representing the predicted blood glucose measurements 120 over the prediction time window (e.g., the next two hours). The mapping performed by the dense layer 450 is constant and not dependent on time. In at least one embodiment, the dense layer 450 takes the form of a fully connected neural network layer with linear activation at each output node.

In at least one embodiment, the encoder 410 has a single layer, having 48 GRU cells, with an internal state dimension of 128. In at least one embodiment, the decoder 420 has a single layer, having 24 GRU cells, with an internal state dimension of 256. In at least one embodiment, tanh is used as the activation function for all RNN layers and sigmoid is used as the recurrent activation function for all RNN layers. In at least one embodiment, during training, L2 regularization is adopted. In at least one embodiment, during training, the lambda of the regularization for all RNN layers is set to 1.7135608462560221e-6. Finally, in at least one embodiment, during training, no dropout or recurrent drop out is used.

It will be appreciated that a GRU cell contains a hidden cell state $h_t$ (i.e., internal state) that is generally passed from one GRU cell to the next in a chain of GRU cells. The GRU cell is configured to selectively add information to its hidden cell state $h_t$ (i.e., remember) or remove information from the hidden cell state $h_t$ (i.e., forget). The addition and/or removal of information is regulated by operation gates of the GRU cell. In a conventional GRU cell, the operation gates include an update gate and a reset gate. First, the update gate includes a sigmoid neural network layer configured to determine how much of the past information needs to be remembered (i.e., added to the hidden cell state $h_t$), based on its input $X_t$ and a previous hidden state $h_{t-1}$, which is also the output of the previous GRU cell in the chain. In contrast, the reset gate includes a sigmoid neural network layer and a hyperbolic tangent (tanh) neural network layer configured to determine how much of the past information needs to be forgotten (i.e., removed from the hidden cell state $h_t$), based on its input $X_t$ and the previous hidden state $h_{t-1}$. The GRU cell determines a new hidden cell state $h_t$ based on the old hidden cell state $h_{t-1}$ and the determinations of the update gate and the reset gate. It will be appreciated that variants of the conventional GRU cell having slightly different operations may also be used.

The neural network layers of the individual GRU cells in the encoder 410 and in the decoder 420 perform their respective functions with reference to a set of weights and/or parameters, which are learned and optimized during a training process. The optimized set of weights and/or parameters are stored in the memory device 274. During usage of the blood glucose management system 200, the processor 276 is configured to execute program instructions corresponding to the blood glucose prediction model 110, 400 with reference to the set of weights and/or parameters stored in the memory device 274. Although the optimal values for the set of weights and/or parameters can be learned during a training process performed by the processor 276, they may be previously generated by another processing system (not shown) beforehand and then stored on the memory device 274. The other processing system may be configured in a conventional manner for a computer or the like having at least a processor and a memory configured to store the training dataset and program instructions for learning the optimized values for the set of weights and/or parameters, which are executed by the processor. In any case, the training of the blood glucose prediction model 400 may be performed using a large number amount of training date, for example including pairs of exemplary input data and ground truth output data

The processor 276 is configured to execute program instructions corresponding to the GRU cells 430 of the encoder 410 with reference to a first set of weights and/or parameters stored on the memory device 274 to determine the encoded vector 440. Similarly, the processor 276 is configured to execute program instructions corresponding to the GRU cells 430 of the decoder 420 with reference to a second set of weights and/or parameters stored on the memory device 274 to determine a decoded output tensor. Finally, the processor 276 is configured to execute program instructions corresponding to the dense layer 450 with reference to a third set of weights and/or parameters stored on the memory device 274 to map the output tensor to the output vector $Y$ representing the predicted blood glucose measurements 120 over the prediction time window (e.g., the next two hours).

Method for Providing Health Information

A variety of operations and processes are described below for operating the blood glucose management system 200 to provide health information to a person 220, and in particular, to predict future blood glucose values of the person 220 based on historical data. In these descriptions, statements that a method, processor, and/or system is performing some task or function refers to a controller or processor (e.g., the processor 218 of the measurement device 210, the processor 260 of the computing device 250, and the processor 272 of the remote server 270) executing programmed instructions stored in non-transitory computer readable storage media (e.g., the memory device 214 of the measurement device 210, the memory device 252 of the computing device 250, and the memory device 274 of the remote server 270) operatively connected to the controller or processor to manipulate data or to operate one or more components in the blood glucose management system 200 to perform the task or function. Additionally, the steps of the methods may be performed in any feasible chronological order, regardless of the order shown in the figures or the order in which the steps are described.

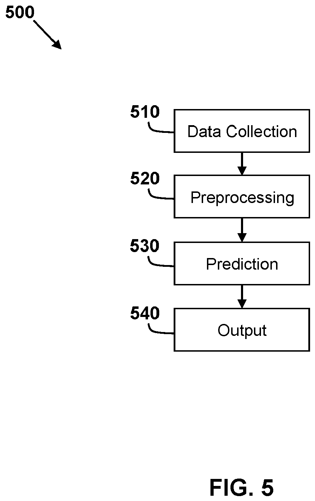

FIG. 5 shows a flow diagram for a method 500 for providing health information. The method 500 advantageously adopts a data preprocessing and feature extraction process for aggregating, distilling, and summarizing the historical blood glucose measurements 130, the insulin intake data 140, and the carbohydrate intake data 150 in the form of an input tensor $X$. An RNN-based blood glucose prediction model 110, 400 is advantageously used to predict future blood glucose measurements 120 of the person 220 based on the input tensor $X$. The predicted future blood glucose measurements 120 are advantageously used to provide a warning of a future health status of the person, as well as to provide a visualization of the predicted future blood glucose measurements.

The method 500 begins with data collection (block 510). Particularly, the blood glucose management system 200 records historical blood glucose measurements 130, insulin bolus dosage data 140, and the carbohydrate intake data 150 for a person over a period of time prior to a cut-off time. In some embodiments, the processor 218 of the measurement device 210 operates the sensor 212 to continuously measure blood glucose concentration values with respect to the person 220. As the blood glucose concentration values are measured over time, they are stored in the memory device 214 of the measurement device 210. Upon request or at some periodic interval, the processor 218 operates the transceiver 216 to transmit these historical blood glucose measurements 130 that are stored in the memory device 214 to one or both of the computing device 250 and the remote server 270. In addition to the continuous glucose monitoring by the measurement device 210, the person 220 inputs the insulin bolus dosage data 140 and the carbohydrate intake data 150 via the input device 256, for example by interacting with a graphical user interface of the glucose management application 262 on the display 258 via the input device 256. However, in other embodiments, the insulin bolus dosage data 140 and the carbohydrate intake data 150 may be determined or measured automatically.

These data 130, 140, and 150 are collected up until a cut-off time, at which a prediction process is triggered. In some embodiments, the processor 272 of the remote server 270 and/or the processor 260 of the computing device 250 may trigger the prediction process automatically on a periodic basis with a predetermined period, for example every 5 minutes. Alternatively, or in addition, the processor 272 of the remote server 270 and/or the processor 260 of the computing device 250 may trigger the prediction process in response to a user input, which may for example be received from the person 220 by interacting with a graphical user interface of the glucose management application 262 on the display 258 via the input device 256.

Once the prediction process is triggered, the historical blood glucose measurements 130, the insulin bolus dosage data 140, and the carbohydrate intake data 150 are transmitted to the remote server 270 for pre-processing and prediction. Particularly, the processor 218 operates the transceiver 216 and/or the processor 260 operates the transceiver 254 to transmit the historical blood glucose measurements 130 to the remote server 270. Likewise, the processor 260 operates the transceiver 254 to transmit the insulin bolus dosage data 140 and the carbohydrate intake data 150 to the remote server 270. The processor 276 operates the transceiver 272 to receive the historical blood glucose measurements 130, the insulin bolus dosage data 140, and the carbohydrate intake data 150 and store them in the memory device 274.

The method 500 continues with preprocessing (block 520). Particularly, the processor 276 of the remote server 270 and/or the processor 260 of the computing device 250 determines a three-dimensional input tensor $X$ for the blood glucose prediction model 110 by preprocessing the historical blood glucose measurements 130, the insulin intake data 140, and the carbohydrate intake data 150. As discussed in greater detail above, the input tensor $X$ includes a plurality of feature vectors $X_i$ corresponding to a sequence of respective time segments $i$ of predetermined length (e.g., 5 minutes). Each feature vector $X_i$ comprises values for a plurality of features for the respective time segment $i$. Thus, the processor 276 and/or the processor 260 calculates the input tensor $X$ by determining, for each respective time segment $i$ from the feature extraction time window, the respective feature vector $X_i$ by determining a respective value for each feature of the plurality of features. Subsequently, the processor 276 stores the input tensor $X$ in the memory device 274, alongside a unique identifier for the person 220 and a timestamp indicating the cut-off time at which the prediction process was triggered.

As discussed in greater detail above, the plurality of features of each feature vector $X_i$ may comprise a variety of different features that aggregate and summarize aspects of the historical blood glucose measurements 130, the insulin intake data 140, and the carbohydrate intake data 150, including the eight features discussed in detail above: "CGM," "CGM AVG_6H," "CGM AVG_1D," "CGM_AGP_14D_Q50_SHIFT_POS_ 264," "BOLUS_INSULIN_ON_BOARD_SHIFT _NEG_24," "UNABSORBED_ CARBS_SHIFT_NEG_24," "TIME_OF_DAY_UNITARY_CIRCLE_COS," and "TIME _OF_DAY_UNITARY_CIRCLE_SIN."

In at least some embodiments, the processor 276 of the remote server 270 executes a general prediction runtime application responsible for performing blood glucose predictions. The general prediction runtime application has a wrapper service that is responsible for preprocessing data prior to prediction. When a request is received by the general prediction runtime application, the wrapper service preprocesses the historical blood glucose measurements 130, the insulin intake data 140, and the carbohydrate intake data 150 to derive the input tensor X, in the manner described above.

The method 500 continues with prediction (block 530). Particularly, the processor 276 of the remote server 270 and/or the processor 260 of the computing device 250 determines predicted future blood glucose measurements 120 of the person based on the input tensor $X$ using the blood glucose prediction model 110. The processor 276 and/or the processor 260 executes the blood glucose prediction model 110 to determine an output tensor, which is mapped to the output vector $Y$ representing the predicted blood glucose measurements 120 over the prediction time window (e.g., the next two hours). In at least some embodiments, the output vector $Y$ includes, not only the predicted blood glucose measurements 120, but also lower/upper error bounds for the prediction. Subsequently, the processor 276 and/or the processor 260 stores the output vector $Y$ in the memory device 274 and/or the memory device 252, alongside the previously stored input tensor $X$, the unique identifier for the person 220, and the timestamp indicating the cut-off. In some embodiments, the processor 276 and/or the processor 260 also stores in the memory device 274 and/or the memory device 252 a log including any errors, warnings, intermediate calculated values, and a version number for the prediction services and/or for the blood glucose prediction model 110.

As noted previously, in the illustrations, the blood glucose prediction model 110 is stored in the memory device 274 of the remote server 270 and is likewise executed by the processor 276 based on data received from the measurement device 110 and from the computing device 250. In other words, the blood glucose prediction model 110 is operated "in the cloud." However, it should be appreciated that in alternative embodiments, the blood glucose prediction model 110 may be stored locally on the memory device 252 of the computing device 250 (e.g., as a part of the glucose management application 262) and executed locally by the processor 260 of the computing device 250. Accordingly, any operations described as being performed by the processor 276 of the remote server 270 should be understood as alternatively being performed locally by the processor 260 of the computing device 250.

After receiving or determining the input tensor $X$, but prior to determining the output vector $Y$ using the blood glucose prediction model 110, the processor 276 and/or the processor 260 first verifies whether the input tensor $X$ fulfills certain technical specifications. In one embodiment, the processor 276 and/or the processor 260 verifies that the input tensor $X$ has the correct dimensions (e.g., [1, 48, 8]). In one embodiment, the processor 276 and/or the processor 260 verifies that all features in the input tensor $X$ are of a correct data type (e.g., decimal float or integer). In one embodiment, the processor 276 and/or the processor 260 verifies that the input tensor $X$ does not contain any null values. In one embodiment, the processor 276 and/or the processor 260 verifies that all features in the input tensor $X$ are within a specified min-max range for the respective feature. If the technical specifications are not fulfilled, the processor 276 and/or the processor 260 generates an error, which is stored in a log in the memory device 274 and/or the memory device 252.

Next, after verifying that the input tensor $X$ fulfills the technical specifications, the processor 276 and/or the processor 260 determines a scaled input tensor $X'$ by scaling values of each respective feature in the plurality of feature vectors $X_i$, for each respective time segment $i$, to fall within a respective predetermined value range for the respective feature (e.g., between 0 and 1). The min-max scalar values are the same across the time dimension but will be different for the different features. In one embodiment, a min-max scaled feature value is determined according to the following equation:

$$x' = \frac{x - \min(x)}{\max(x) - \min(x)}$$

where $x$ is the original feature value, $x'$ is the scaled feature value, $\max(x)$ is a maximum value for the feature, and $\min(x)$ is a minimum value for the feature. If the scaling cannot be performed, the processor 276 and/or the processor 260 generates an error, which is stored in a log in the memory device 274 and/or the memory device 252.

After the scaled input tensor $X'$ is determined, the processor 276 and/or the processor 260 executes the encoder and decoder layers of the blood glucose prediction model 110 to determine an output tensor and the dense layer of the blood glucose prediction model 110 to map the output tensor to an initial output vector $Y'$. As discussed previously, in at least some embodiments, the blood glucose prediction model 110 expects the plurality of feature vectors $X_i$ in the input tensor $X$ to be organized in the time dimension from oldest to most recent. Additionally, in some embodiments, the blood glucose prediction model 110 expects the feature values to be organized in the feature dimension in a particular order (e.g., alphabetical according to feature name). The processor 276 and/or the processor 260 verifies that the feature vectors $X_i$ and the feature values thereof are correctly ordered. If not, the processor 276 and/or the processor 260 attempts to reorder the feature vectors $X_i$ and the feature values if possible. If the model prediction cannot be triggered, the processor 276 and/or the processor 260 generates an error, which is stored in a log in the memory device 274 and/or the memory device 252.

After the initial output vector $Y'$ is determined, the processor 276 and/or the processor 260 determines a rescaled output vector $Y$ by rescaling the initial output vector $Y'$ using an inverse of the process that was used to determine the scaled input tensor $X'$ from the original input tensor $X$. Particularly, the initial output vector $Y'$ is rescaled to fall between minimum and maximum values for the prediction (predicted blood glucose measurements), rather than the predetermine range for the prediction provided by the blood glucose prediction model 110. The min-max scalar values are the same across the time dimension. In one embodiment, each rescaled prediction is determined according to the following equation:

$$y = y'(\max(y) - \min(y)) + \min(y)$$

where $y'$ is the originally output prediction value, $y$ is the rescaled output prediction value (predicted blood glucose measurement), $\max(y)$ is a maximum value for the prediction (e.g., a maximum blood glucose measurement value), and $\min(y)$ is a minimum value for the prediction (e.g., a minimum blood glucose measurement value). If the rescaling cannot be performed, the processor 276 and/or the processor 260 generates an error, which is stored in a log in the memory device 274 and/or the memory device 252.

After the rescaled output vector $Y$ is determined, the processor 276 and/or the processor 260 performs a calibration or bias correction with respect to the output vector $Y$. Particularly, the processor 276 and/or the processor 260 determines a calibrated output vector $Y_{pred}$ including calibrated predicted blood glucose measurements $y_{pred}$ by adding an estimated bias value $b$ to each of the predicted blood glucose measurements $y$ in the rescaled output vector $Y$. In one embodiment, each calibrated predicted blood glucose measurement is determined according to the following equation:

$$f_j(p_j) = p_j + b_j$$

where $p$ is the prediction value (predicted blood glucose measurement), $b$ is an estimated bias value, $f(p)$ is the recalibrated prediction, and $j$ indicates a prediction time horizon (e.g., between 5 minutes and 120 minutes into the future). In at least some embodiments, each bias value $b$ is different depending on the prediction time horizon $j$. For example, the bias value $b$ may be smaller for a relatively shorter prediction time horizon $j$ and larger for a relatively longer prediction time horizon $j$. If the calibration cannot be performed, the processor 276 and/or the processor 260 generates an error, which is stored in a log in the memory device 274 and/or the memory device 252.

After the calibrated output vector $Y_{pred}$ is determined, the processor 276 and/or the processor 260 determines an upper error bound $ue_t$ and lower error bound $le_t$. Particularly, for each prediction time horizon $t$ (each time segment of the prediction time window), the processor 276 and/or the processor 260 determines a respective error bar width $\Delta_t$. In one embodiment, the size of the error bounds is selected to provide a coverage of 50%. In at least one embodiment, the error bar width $\Delta_t$ for each prediction time horizon $t$ is determined according to the following equation:

$$\Delta_t = \begin{cases} \dfrac{v_t}{100} * y_{pred_t} & for\ y_{pred_t} > 100mg/dL \\ v_t & for\ y_{pred_t} \leq 100mg/dL \end{cases}$$

where $t$ is the prediction time horizon, $\Delta_t$ is the error bar width at the time t, $y_{pred_t}$ is the bias corrected (calibrated) prediction at the time $t$, and $v_t$ is a predetermined value for the time $t$. In one embodiment, the value $v_t$ for each prediction time horizon is stored in a look-up table stored in the memory device 274 and/or the memory device 252. The processor 276 and/or the processor 260 determines the upper error bound $ue_t$ and the lower error bound $le_t$ for each prediction time horizon $t$ based on the calibrated prediction $y_{predt}$ and the error bar width $\Delta_t$ according to the following equations:

$$ue_t = y_{pred_t} + \Delta_t$$

$$le_t = y_{pred_t} - \Delta_t$$

After the upper error bounds $ue_t$ and lower error bounds $le_t$ are determined, the processor 276 and/or the processor 260 forms an upper error bound vector $UE$ with all of the upper error bounds $ue_t$ and a lower error bound vector $LE$ with all of the lower error bounds $le_t$. The processor 276 and/or the processor 260 determines a final output tensor by concatenating the calibrated output vector $Y_{pred}$ with the lower error bound vector $LE$ and the upper error bound vector $UE$.

Finally, the processor 276 and/or the processor 260 verifies whether the final output tensor fulfills certain technical specifications. In one embodiment, the processor 276 and/or the processor 260 verifies that the final output tensor has the correct dimensions (e.g., [1, 24, 3]). In one embodiment, the processor 276 and/or the processor 260 verifies that the final output tensor does not contain any null values. In one embodiment, the processor 276 and/or the processor 260 verifies that the final output tensor includes float values with a predetermined number of decimals (e.g., 2 decimals). In one embodiment, the processor 276 and/or the processor 260 verifies that component vectors of the final output tensor are ordered correctly (e.g., (1) the calibrated output vector $Y_{pred}$, (2) the lower error bound vector $LE$, and (3) the upper error bound vector $UE$). In one embodiment, the processor 276 and/or the processor 260 verifies that predictions and error bounds in the final output tensor are ordered correctly in the time dimension (e.g., starting with values on the shortest prediction time horizon - 5 minutes - and ending with the values on the longest prediction time horizon - 120 minutes). Finally, the processor 276 and/or the processor 260 attaches a version number for the prediction services and/or for the blood glucose prediction model 110, or confirms that it was already attached. If the technical specifications are not fulfilled, the processor 276 and/or the processor 260 generates an error, which is stored in a log in the memory device 274 and/or the memory device 252. Otherwise, the processor 276 and/or the processor 260 stores the final output tensor in the memory device 274 and/or the memory device 252.

In at least some embodiments, the processor 276 and/or the processor 260 executes a general prediction pipeline of the general prediction runtime application to perform the operations described above. The general prediction pipeline may, for example, comprise a Python script that utilizes tensorflow and h5py libraries. After determining the final output tensor, the processor 276 returns the final output tensor to the wrapper service.

The method 500 continues with output (block 540). Particularly, the computing device 250 displays to a user (e.g., the person 220) a visualization of the predicted future blood glucose measurements or a warning of a possible future health status. More particularly, in some embodiments, the processor 276 of the remote server 270 operates the transceiver 272 to transmit the final output tensor to the computing device 250. The processor 260 of the computing device 250 operates the transceiver 254 to receive the final output tensor and then stores the final output tensor in the memory device 252. The computing device 250 may be configured to provide a wide variety of useful and advantageous features using the final output tensor, which in includes the predicted future blood glucose measurements 120. Two such useful features include (1) displaying a visualization of the predicted future blood glucose measurements 120 to the person 220, such as a data plot and (2) providing an early perceptible warning of a predicted health status of the person 220, such as a predicted or current hypoglycemic condition or hyperglycemic condition of the person 220.

In a first example, the processor 260 operates the display 258 to display a data plot showing the historical blood glucose measurements 130 and/or the predicted future blood glucose measurements 120. In some embodiments, a data plot may further show the insulin bolus dosage data 140 and the carbohydrate intake data 150. Such a data plot advantageously enables the person 220 to more easily and better understand their health and how to better manage their blood glucose concentration throughout the day. The predicted future blood glucose measurements 120 can help the person 220 to better appreciate the relationships that time of day, insulin bolus dosing, and the carbohydrate intake can have on his or her blood glucose concentration. Additionally, in at least some embodiments, such data plots also provide a visualization of the upper and lower error bounds, for example by surrounding the blood glucose concentration data on the data plot with error bands corresponding to the upper and lower error bounds at each prediction time horizon. This advantageously helps the person 220 to better understand the uncertainty in the predicted future blood glucose measurements 120.

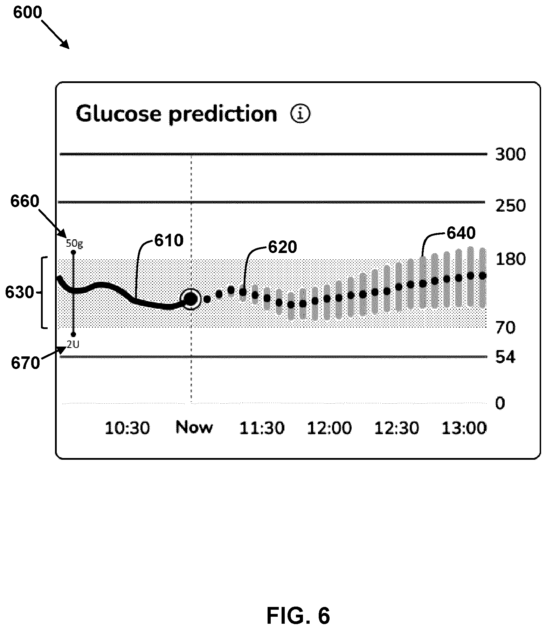

FIG. 6 shows a portion of a graphical user interface including data plot 600 that visualizes the both the historical blood glucose measurements 130 and/or the predicted future blood glucose measurements 120. Particularly, the data plot 600 includes a solid-line curve 610 that represents a subset of the historical blood glucose measurements 130 that were measured immediately prior to the current time. Similarly, the data plot 600 includes a dotted-line curve 620 that represents the predicted future blood glucose measurements 120 and extends two hours into the future. The solid-line curve 610 and the dotted-line curve 620 are superimposed upon a vertical band 630, which is shaded to indicate a range of ideal blood glucose measurement values (e.g., between 70 mg/dL and 180 mg/dL) in which the person 220 is not considered to be in a hypoglycemic or hyperglycemic condition. Additionally, each prediction the dotted-line curve 620 (indicated by an individual dot) is paired with an error band 640, which is shaded to represent the upper and lower error bounds for the particular prediction. Finally, the data plot 600 includes data annotations 660 and 670, which indicate amounts of carbohydrates consumed (e.g., "50g") and amounts of insulin intake (e.g., "2U"), respectively.

In some embodiments, there are certain circumstances in which the predicted future blood glucose measurements 120 themselves are not displayed to the person 220. In one embodiment, the processor 260 determines whether the person 220 is currently in a hypoglycemic condition based on the last reported blood glucose measurement from the measurement device or in the previously provided input tensor $X$. If the user is in a hypoglycemic condition, no prediction is shown to the person 220. Instead of the prediction, a warning message is shown on the display 258. In another example, when a hypoglycemic condition is predicted at some point in the next 2 hours, prediction values after the start of the hypoglycemic condition are not shown to the person 220. The processor 260 identifies, in the predicted future blood glucose measurements 120, a first predicted value that is less than a predetermined low glucose alarm limit (e.g., below 70 mg/dL) and all predicted values after the identified first predicted value are removed and not shown to the person 220. If the Short-term Hypoglycemia Prediction (STHP) is currently at risk state 1 (high risk) and the predicted future blood glucose measurements 120 do not predict a hypoglycemic condition in the next 30 minutes, defined as all lower bound values within the next 30 minutes being greater than or equal to the predetermined low glucose alarm limit, then the prediction is not displayed. Otherwise, the processor 260 identifies in the predicted future blood glucose measurements 120 a first predicted value within the next 30 minutes that is less than a predetermined low glucose alarm limit and all predicted values after the identified first predicted value are removed and not shown to the person 220.

In at least some embodiments, the processor 260 of the computing device 250 is configured to determine, based on the predicted future blood glucose measurements 120, whether a possible hypoglycemic condition of the person 220 or a possible hyperglycemic condition of the person 220 is likely within the prediction time window. In one embodiment, the processor 260 determines the possible hypoglycemic condition of the person 220 may occur within the prediction time window in response to any of the predicted future blood glucose measurements 120 being less than the predetermined low glucose alarm limit. In one embodiment, the processor 260 determines the possible hyperglycemic condition of the person 220 may occur within the prediction time window in response to any of the predicted future blood glucose measurements 120 being greater than a predetermined high glucose alarm limit. In some embodiments, the whether the possible hypoglycemic condition of the person 220 or the possible hyperglycemic condition of the person 220 is likely within the prediction time window using a machine learning model, which is separate from the blood glucose prediction model 110, 400.

In response to determining that the possible hypoglycemic condition of the person 220 or the possible hyperglycemic condition of the person 220 is likely within the prediction time window, the processor 260 operates the display 258 or other output devices to output a perceptible alert or warning. The visual alert is a typical smartphone notification or any other image and/or text that informs the person 220 of the possible hyperglycemic or hypoglycemic condition. Additionally, or alternatively, the computing device 250 generates an auditory alert using a speaker (not shown). The auditory alert is a sound that the person 220 associates with the possible hyperglycemic or hypoglycemic condition, such a typical smartphone notification sound or any other brief sound. Additionally, or alternatively, the computing device 250 generates a tactile alert using a haptic actuator (not shown). The tactile alert causes the computing device 250 to vibrate thereby informing the person 220 of the possible hyperglycemic or hypoglycemic condition. The computing device 250 may be additionally or alternatively configured to inform the person 220 of the possible hyperglycemic or hypoglycemic condition using any other notification method, such as by sending the person 220 a text message, sending the person 220 an email, and/or by making an automated phone call to the person 220. The computing device 250, additionally or alternatively, provides the same types of alerts and/or notifications to a caretaker of the person, such as a nurse or the like.

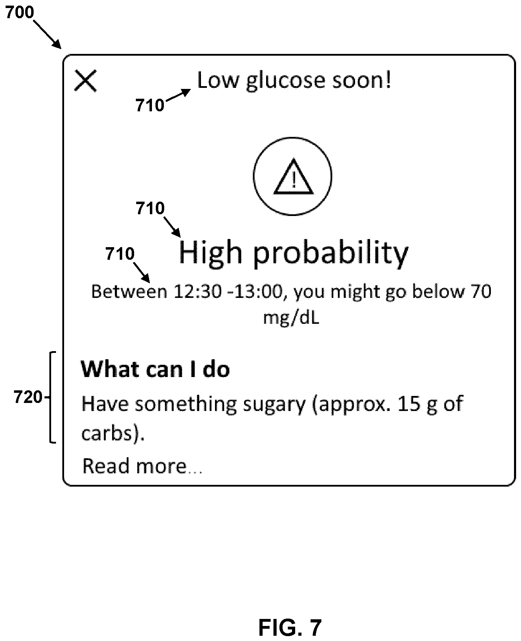

FIG. 7 shows a portion of a graphical user interface including visual alert 700 that lets a user know that his or her blood glucose has a high probability of falling below a threshold value. Particularly, the visual alert 700 is a typical smartphone notification including text 710 (e.g., "Low glucose soon!" "High probability" and "Between 12:30 - 13:00, you might go below 70 mg mg/dL") that informs the person 220 of the possible hypoglycemic condition. In addition, the visual alert 700 include additional text 720 (e.g., "What can I do" and "Have something sugary (approx. 15 g of carbs).") that informs the person 220 of a recommended action that can be taken to avoid the possible hypoglycemic condition.

In response to receiving the alert and/or the notification regarding a possible hyperglycemic condition, the person 220 may want to provide themselves with a bolus dose of the medicament 232 from the administration device 230. In some embodiments, the processor 260 may determine, and display on the display 258, information concerning a recommended dosage amount of the medicament 232 that should be dosed to the person 220 to attempt to prevent the hyperglycemic condition that was predicted in the predicted future blood glucose measurements 120.

For certain types of administration devices 230, the computing device 250 is configured to transmit the bolus advisor data to the administration device 230 using the transceiver 254, so that the administration device 230 can deliver a desired dosage of the medicament 232 to the person 220. The amount of the medicament 232 that is delivered is based on the historical blood glucose measurements 130 and/or the predicted future blood glucose measurements 120, with the objective being to reduce the glucose concentration level of the person 220 to a normal or desired level. In an example, the administration device 230 is an insulin pump that is configured to automatically deliver a bolus dose of insulin to the person 220. In another example, the administration device 230 is a smart insulin pen that is configured to deliver a bolus dose for injection. In each example, the amount of the insulin that is delivered in the bolus dose is based on the historical blood glucose measurements 130 and/or the predicted future blood glucose measurements 120.

Embodiments within the scope of the disclosure may also include non-transitory computer-readable storage media or machine-readable medium for carrying or having computer-executable instructions (also referred to as program instructions) or data structures stored thereon. Such non-transitory computer-readable storage media or machine-readable medium may be any available media that can be accessed by a general purpose or special purpose computer. By way of example, and not limitation, such non-transitory computer-readable storage media or machine-readable medium can comprise RAM, ROM, EEPROM, CD-ROM or other optical disk storage, magnetic disk storage or other magnetic storage devices, or any other medium which can be used to carry or store desired program code means in the form of computer-executable instructions or data structures. Combinations of the above should also be included within the scope of the non-transitory computer-readable storage media or machine-readable medium.

Computer-executable instructions include, for example, instructions and data which cause a general-purpose computer, special purpose computer, or special purpose processing device to perform a certain function or group of functions. Computer-executable instructions also include program modules that are executed by computers in stand-alone or network environments. Generally, program modules include routines, programs, objects, components, and data structures, etc. that perform particular tasks or implement particular abstract data types. Computer-executable instructions, associated data structures, and program modules represent examples of the program code means for executing steps of the methods disclosed herein. The particular sequence of such executable instructions or associated data structures represents examples of corresponding acts for implementing the functions described in such steps.

While the disclosure has been illustrated and described in detail in the drawings and foregoing description, the same should be considered as illustrative and not restrictive in character. It is understood that only the preferred embodiments have been presented and that all changes, modifications and further applications that come within the spirit of the disclosure are desired to be protected.

## Claims

1.    A method for providing health information, the method comprising:

receiving, with a processor, historical blood glucose measurements of a person measured prior to a cut-off time;
determining, with the processor, an input tensor comprising a plurality of feature vectors based on the historical blood glucose measurements, each respective feature vector corresponding to a respective time segment from a first time window that ends prior to the cut-off time;

determining, with the processor, based on the input tensor using a machine learning model, predicted blood glucose measurements of the person for a second window of time that starts after the cut-off time; and

providing at least one of (i) a visualization of the predicted future blood glucose measurements on a display of an electronic device and (ii) a perceptible warning of a possible future health status via the electronic device.

2. The method according to claim 1, the determining the input tensor further comprising:

determining, for each respective time segment from the first time window, the respective feature vector by determining a respective value for each feature of a plurality of features.

3. The method according to claim 2, the determining the input tensor further comprising:

calculating, for each respective time segment from the first time window, as one of the plurality of features of the respective feature vector, an average blood glucose measurement of the person calculated over a predetermined duration of time ending at an end of the respective time segment.

4. The method according to claim 2, the determining the input tensor further comprising:

determining, for each respective time segment from the first time window, as one of the plurality of features of the respective feature vector, an average blood glucose measurement of the person calculated over a plurality of previous time segments of a plurality of previous days that are a same time of day as the respective time segment.

5. The method according to claim 2, the determining the input tensor further comprising:

determining, for each respective time segment from the first time window, as one of the plurality of features of the respective feature vector, an average blood glucose measurement of the person calculated over a plurality of previous time segments of a plurality of previous days that are a same time of day as the respective time segment, shifted forward in time by a predetermined amount of time.

6. The method according to claim 2 further comprising:

receiving, with the processor, insulin intake data indicating insulin doses taken by the person prior to the cut-off time,

wherein the input tensor is determined based on the historical blood glucose measurements and the insulin intake data,

wherein the determining the input tensor further comprising:

calculating, for each respective time segment from the first time window, as one of the plurality of features of the respective feature vector, using an insulin absorption model, an estimated insulin on board of the person at a respective time that is a predetermined amount of time after an end of the respective time segment, the respective time being subsequent to the cut-off time.

7. The method according to claim 2 further comprising:

receiving, with the processor, carbohydrate consumption data indicating carbohydrates consumed by the person prior to the cut-off time,

wherein the input tensor is determined based on the historical blood glucose measurements and the carbohydrate consumption data.

8. The method according to claim 7, the determining the input tensor further comprising:

calculating, for each respective time segment from the first time window, as one of the plurality of features of the respective feature vector, using a carbohydrate absorption model, an estimated amount of unabsorbed carbohydrates in the person at a respective time that is a predetermined amount of time after an end of the respective time segment, the respective time being subsequent to the cut-off time.

9. The method according to claim 2, the determining the input tensor further comprising:

calculating, for each respective time segment from the first time window, a respective normalized time of day based on a time of day of an end of the respective time segment, the respective normalized time of day being a value between zero and two-pi radians; and

calculating, as at least one of the plurality of features of the respective feature vector, at least one of (i) a cosine of the normalized time of day and (ii) a sine of the normalized time of day.

10. The method according to any one of the preceding claims, wherein the machine learning model includes a recurrent neural network having an encoder-decoder architecture, an encoder of the recurrent neural network having a first plurality of gated recurrent unit cells and a decoder of the recurrent neural network having a second plurality of gated recurrent unit cells.

11. The method according to any one of the preceding claims, the determining the predicted blood glucose measurements further comprising:

determining a scaled input tensor by scaling values of each respective feature in the plurality of feature vectors to fall within a respective predetermined range for the respective feature; and
determining the predicted blood glucose measurements based on the scaled input tensor using the machine learning model.

12. The method according to any one of the preceding claims, the determining the predicted blood glucose measurements further comprising:

determining an output tensor using the machine learning model; and
mapping the output tensor to an output vector including the predicted blood glucose measurements,
wherein the determining the predicted blood glucose measurements further comprising:

determining a scaled output vector including scaled predicted blood glucose measurements by scaling the output vector such that the scaled predicted blood glucose measurements fall between a minimum blood glucose measurement and a maximum blood glucose measurement, and/or
wherein the determining the predicted blood glucose measurements further comprising:
determining a calibrated output vector including calibrated predicted blood glucose measurements by adding a bias value to each of the predicted blood glucose measurements.

13. The method according to any one of the preceding claims, the determining the predicted blood glucose measurements further comprising:

determining, for each predicted blood glucose measurements, a respective upper error bound and a respective lower error bound,
wherein the providing the visualization further comprising:
displaying, on the display of the electronic device, a visualization of each of the predicted future blood glucose measurements in association with the respective upper error bound and the respective lower error bound.

14. The method according to any one of the preceding claims, the providing the perceptible warning further comprising:

determining that the predicted future blood glucose measurements indicate one of (i) a possible hyperglycemic condition of the person and (ii) a possible hypoglycemic condition of the person; and
outputting, via an output device of the electronic device, the perceptible warning in response to determining that the predicted future blood glucose measurements indicate the one of (i) the possible hyperglycemic condition of the person and (ii) the possible hypoglycemic condition of the person.

15. A system for providing health information, the system comprising:

a memory device;
at least one output device;
a processor operably connected to the memory device and the least one output device, the processor being configured to:

receive, and store in the memory device, historical blood glucose measurements of a person measured prior to a cut-off time;
determine an input tensor comprising a plurality of feature vectors based on the historical blood glucose measurements, each respective feature vector corresponding to a respective time segment from a first time window that ends prior to the cut-off time;

determine, based on the input tensor using a machine learning model, predicted blood glucose measurements of the person for a second window of time that starts after the cut-off time; and
operate the at least one output device to at least one of (i) display a visualization of the predicted future blood glucose measurements and (ii) output a perceptible warning of a possible future health status.

**130**

Blood Glucose
Measurements

**140**

Insulin Bolus Dosage
Data

**150**

Carbohydrate Intake
Data

**110**

Blood Glucose
Prediction Model

Predicted Future
Blood Glucose
Measurements

**120**

**160**

Visualizations

**170**

Early
Warnings

# FIG. 1

FIG. 2

FIG. 3

EP 4 610 994 A1

FIG. 4

**500**

**510** | Data Collection

**520** | Preprocessing

**530** | Prediction

**540** | Output

# FIG. 5

**FIG. 6**

700

× Low glucose soon!

710

⚠

710

710 High probability

Between 12:30 -13:00, you might go below 70 mg/dL

720

**What can I do**

Have something sugary (approx. 15 g of carbs).

Read more...

**FIG. 7**

Europäisches
Patentamt

European
Patent Office

Office européen
des brevets

# EUROPEAN SEARCH REPORT

**Application Number**

EP 24 16 0294

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/375549 A1 (WEXLER YDO [IL] ET AL) 3 December 2020 (2020-12-03) | 1-5,7, 9-15 | INV. G16H20/17 G16H50/20 |
| Y | * [0003], [0014], [0049], [0057], [0059], [0061], [0063], [0069], [0074], [0117], [0118], figures 6c-6f * | 6,8 | |
| Y | SAHIN ASIYE ET AL: "Personalized Advanced Time Blood Glucose Level Prediction", ARABIAN JOURNAL FOR SCIENCE AND ENGINEERING, SPRINGER BERLIN HEIDELBERG, BERLIN/HEIDELBERG, vol. 46, no. 10, 5 January 2021 (2021-01-05), pages 9333-9344, XP037561713, ISSN: 2193-567X, DOI: 10.1007/S13369-020-05263-2 [retrieved on 2021-01-05] | 6,8 | |
| A | * abstract, 2.6 predicting of ..., 3.2.1 insulin on board model, 3.2.2 glucose absorption rate model, 5 conclusions * | 1-5,7, 9-15 | |
| A | KILIAN MERKELBACH: "Novel architecture for gated recurrent unit autoencoder trained on time series from electronic health records enables detection of ICU patient subgroups", SCIENTIFIC REPORTS, vol. 13, no. 1, 11 March 2023 (2023-03-11), XP093167337, US ISSN: 2045-2322, DOI: 10.1038/s41598-023-30986-1 Retrieved from the Internet: URL:https://www.nature.com/articles/s41598-023-30986-1.pdf> * abstract; p.17, model * | 1-15 | TECHNICAL FIELDS SEARCHED (IPC) G16H |

-/--

The present search report has been drawn up for all claims

| Place of search | Date of completion of the search | Examiner |
|---|---|---|
| Munich | 28 May 2024 | Sundqvist, Benjamin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 16 0294

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2021/375448 A1 (DERDZINSKI MARK [US] ET AL) 2 December 2021 (2021-12-02) * [0107], [0108] * | 1-15 | |
| A | US 2023/044319 A1 (NEEMUCHWALA HUZEFA F [US] ET AL) 9 February 2023 (2023-02-09) * [0010], [0122], [0125], [0149] * | 1-15 | |
| A | Ananth Bhimireddy: "Blood Glucose Level Prediction as Time-Series Modeling using Sequence-to-Sequence Neural Networks", , 30 August 2020 (2020-08-30), XP093167043, Retrieved from the Internet: URL:https://ceur-ws.org/Vol-2675/paper22.pdf * 3.2.5 description of ..., 5 conclusion and ... * | 1-15 | |

TECHNICAL FIELDS
SEARCHED        (IPC)

The present search report has been drawn up for all claims

| Place of search | Date of completion of the search | Examiner |
|---|---|---|
| Munich | 28 May 2024 | Sundqvist, Benjamin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 16 0294

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2024

| Patent document cited in search report | Publication date | Patent family member(s) | | Publication date |
|---|---|---|---|---|
| US 2020375549 A1 | 03-12-2020 | AU | 2020283127 A1 | 20-01-2022 |
| | | CA | 3142003 A1 | 03-12-2020 |
| | | CN | 114207737 A | 18-03-2022 |
| | | EP | 3977480 A1 | 06-04-2022 |
| | | JP | 2022534422 A | 29-07-2022 |
| | | KR | 20220016487 A | 09-02-2022 |
| | | US | 2020375549 A1 | 03-12-2020 |
| | | WO | 2020243576 A1 | 03-12-2020 |
| US 2021375448 A1 | 02-12-2021 | AU | 2020450952 A1 | 10-11-2022 |
| | | CA | 3176599 A1 | 02-12-2021 |
| | | CN | 115552242 A | 30-12-2022 |
| | | EP | 4158335 A1 | 05-04-2023 |
| | | JP | 2023529547 A | 11-07-2023 |
| | | US | 2021369151 A1 | 02-12-2021 |
| | | US | 2021375447 A1 | 02-12-2021 |
| | | US | 2021375448 A1 | 02-12-2021 |
| | | WO | 2021242304 A1 | 02-12-2021 |
| US 2023044319 A1 | 09-02-2023 | CA | 3055770 A1 | 27-09-2018 |
| | | CN | 110650683 A | 03-01-2020 |
| | | CN | 114983403 A | 02-09-2022 |
| | | EP | 3600036 A1 | 05-02-2020 |
| | | US | 2018271455 A1 | 27-09-2018 |
| | | US | 2018272063 A1 | 27-09-2018 |
| | | US | 2018272064 A1 | 27-09-2018 |
| | | US | 2018272065 A1 | 27-09-2018 |
| | | US | 2018272066 A1 | 27-09-2018 |
| | | US | 2018277242 A1 | 27-09-2018 |
| | | US | 2018277246 A1 | 27-09-2018 |
| | | US | 2021259641 A1 | 26-08-2021 |
| | | US | 2022096021 A1 | 31-03-2022 |
| | | US | 2023044319 A1 | 09-02-2023 |
| | | WO | 2018175935 A1 | 27-09-2018 |

EPO FORM P0459

For more details about this annex : see Official Journal of the European Patent Office, No. 12/82